Europäisches Patentamt

European Patent Office

Office européen des brevets

(19)

(11) Publication number: **0 332 175**
**A2**

(12)

# EUROPEAN PATENT APPLICATION

(21) Application number: 89104123.8

(22) Date of filing: 08.03.89

(51) Int. Cl.⁴: **B01J 13/02**

(30) Priority: 10.03.88 JP 57894/88

(43) Date of publication of application:
**13.09.89 Bulletin 89/37**

(84) Designated Contracting States:
**AT BE CH DE ES FR GB GR IT LI LU NL SE**

(71) Applicant: **LION CORPORATION**
**3-7, Honjo 1-chome**
**Sumida-ku Tokyo(JP)**

(72) Inventor: **Takizawa, Masahiro**
**4786, Kusu Kawasato-mura**
**Kitasaitama-gun Saitama(JP)**
Inventor: **Matsui, Yumiko**
**3-17-14, Honcho**
**Kokubunji-shi Tokyo(JP)**
Inventor: **Arai, Hiroto**
**4-15-6-611, Sekimachiminami**
**Nerima-ku Tokyo(JP)**

(74) Representative: **Henkel, Feiler, Hänzel &**
**Partner**
**Möhlstrasse 37**
**D-8000 München 80(DE)**

(54) **Method of producing microcapsule.**

(57) A method of producing microcapsules comprising the steps of:
mixing (i) an aqueous dispersion containing a capsule having an wall membrane containing polyvinyl alcohol and an aqueous solution containing 8% to 50% by weight of an electrolyte or electrolytes and (ii) an aldehyde or aldehydes having a solubility in water at 20° C of not more than 10%; and
allowing the mixture to react under an acidic condition.

EP 0 332 175 A2

## METHOD OF PRODUCING MICROCAPSULE

BACKGROUND OF THE INVENTION

1. Field of the Invention

The present invention relates to a method of producing a microcapsule capable of protecting a core substance by completely blocking a permeation of foreign materials, and having a high safety factor with respect to a human body.

2. Description of the Related Art

Microcapsules have excellent functions in that liquids and gases can be treated as a solid; substances which react with each other can be isolated; and the bleeding of core substances can be controlled in accordance with changes in an ambient environment.

The following techniques for producing dense capsules having an excellent core substance retentionability have been reported.

(1) Japanese Examined Patent Publication (Kokoku) No. 46-38244 discloses a method of treating a capsule wall membrane such as gelatin with a cross-linking agent such as formaldehyde and glutaraldehyde, followed by drying and heating at a temperature of 120°C to 150°C.

(2) GB Patent No. 1190721 discloses a method of adding a polyhydroxy aromatic substance such as resorcinol, hydroquinone, and catechol to a dispersion containing a capsule having a wall membrane of a hydrophilic polymer, such as polyvinyl alcohol, gelatin, and methyl cellulose, which membrane is swollen with a solvent to effect a reaction of the polyhydroxy aromatic substance with the wall membrane, followed by reacting the polyhydroxy aromatic substance with an aldehyde such as formaldehyde, glyoxal, furfural and glutaraldehyde.

(3) Japanese Unexamined Patent Publication (Kokai) No. 56-100630 discloses a method of treating an aqueous dispersion containing a capsule having a wall membrane of polyvinyl alcohol with, for example, acids and dialdehydes such as glyoxal and glutaraldehyde; or acids and urea and/or melamine and formaldehyde; or alkali and divinylsulfone, methylvinyl ketone; or alkali and an epichlorohydrin compound.

(4) Japanese Unexamined Patent Publication (Kokai) No. 56-100631 discloses a method of treating an aqueous dispersion containing a capsule having a wall membrane of polyvinyl alcohol with an organic titanium compound such as diisopropoxy titanium bisacetyl acetonate and aminoalcohol titanium chelate.

(5) U.S. Patent No. 3630955 discloses a method of hydrophobically treating an aqueous dispersion containing a capsule having a wall membrane of a hydrophilic polymer substance, by a free radical polymerizing of a polymerizable unsaturated monomer having a nitrile group and the wall membrane substance in the presence of, for example, iron, cobalt, nickel, copper, chromium, uranyl, and vanadium.

The above-mentioned methods, however, are used to make a water-soluble polymer substantially water-insoluble, to obtain a wall membrane of a capsule by, for example, a crosslinking or chelating reaction with an ion, and thus a large number of hydrophilic groups is present in the polymer constituting a wall membrane of a capsule. For this reason, although a wall membrane of a capsule exhibits a high impermeability in a system having a low water content, the wall membrane absorbs water and is swollen in an aqueous solution system and, therefore, a core substance having even a slight water-solubility will gradually permeate through the wall membrane. Especially, when a surfactant having a strong permeability is present, the permeability through the wall membrane is remarkably increased and even a water-insoluble core substance is disadvantageously ejected from the capsule. Furthermore, a treatment agent having a strong toxicity is sometimes used as a hardening agent for the impermeable treatment.

On the other hand, when a microcapsule having a wall membrane of polyvinyl alcohol is produced by a simple coacervation method, a uniform coating cannot be obtained and non-coated portions are generated, depending upon the core substance, and an aggregation of the capsules occurs whereby acceptable capsules cannot be obtained. These phenomena occur most often, when hydrophobic substances having a polar group, for example, alcohols such as hexanol linalool, benzyl alcohol, and eugenol; and aldehydes such as trans-2-hexanal, benzaldehyde, and anise aldehyde, are used.

SUMMARY OF THE INVENTION

Accordingly, the object of the present invention is to eliminate the above-mentioned disadvantages of the prior art and to provide a method of producing a microcapsule capable of providing a high degree of protection of a core substance and of completely blocking the permeability of a core substance in an aqueous solution, especially even in an aqueous solution containing a surfactant.

Other objects and advantages of the present invention will be apparent from the following description.

In accordance with the present invention, there is provided a method of producing microcapsules comprising the steps of:

mixing (i) an aqueous dispersion containing a capsule having a wall membrane containing polyvinyl alcohol, and an aqueous solution containing 8% to 50% by weight of an electrolyte or electrolytes, and (ii) an aldehyde or aldehydes having a solubility in water at 20° C of not more than 10%; and

allowing the mixture to react under an acidic condition.

DESCRIPTION OF THE PREFERRED EMBODIMENTS

As a method of producing a microcapsule having a wall membrane containing polyvinyl alcohol, a salt coacervation method (U.S. Patent Nos. 3574133, 3565818, and 4777089 and Japanese Unexamined Patent Publication No. 62-7440), a phase separation method utilizing a cloud point (U.S. Patent No. 4269729), a spray drying method, and a hardening-in-liquid method can be used. Of these methods, a salt coacervation method (a simple coacervation method) is preferable; in particular, a method in which a substantially water insoluble core substance is dispersed in an aqueous solution containing polyvinyl alcohol, as a wall membrane forming substance, while an electrolyte is added thereto. Furthermore, preferably the pectin is added simultaneously with or after the addition of the electrolyte. The pectin can be added in the form of a powder, but preferably, is added as an aqueous solution. Optionally, the pectin is added by dissolving it in an aqueous electrolyte solution. More preferably, the pectin is previously dissolved at a concentration of 0.05% to 0.5% by weight in water and, after the core substance is dispersed in water, the aqueous pectin solution is added thereto simultaneously with the electrolyte or after the addition of the electrolyte. When a core substance is apt to cause aggregation, preferably the pectin is added simultaneously with the addition of the electrolyte. If a necessary amount of the pectin is added before the addition of the electrolyte, the wall membrane of a microcapsule will be very thin or the wall membrane will not be formed. Preferably, the pectin is added in an amount of 0.1% to 1.0% by weight, based on the amount of water.

When the above-mentioned method is adopted, an acceptable microcapsule can be produced while preventing the formation of a weak membrane or an aggregation of the microcapsules, even when the above-mentioned hydrophobic core substances having a polar group are used.

The polyvinyl alcohol usable in the present invention preferably contains at least 50% by weight of a vinyl alcohol component in the polymer. In addition to the polymer (homopolymer in which all the polymers consist of vinyl alcohol units, polymers with 50% by weight or more of vinyl alcohol component containing other components such as vinyl acetate, vinyl propionate, vinyl butyrate, etc., and anionically modified products thereof or cationically modified products thereof are also included in the polyvinyl alcohol as mentioned in the present invention.

Polyvinyl alcohols are generally available as hydrolyzates of polyvinyl acetates, preferably having a 70 to 100 mole% hydrolysis ratio. Also, two or more polyvinyl alcohols with different hydrolysis ratios may be used as a mixture. The concentration of the polyvinyl alcohol in an aqueous solution is preferably 0.5 to 15% by weight, more preferably 1 to 10% by weight.

When water-soluble polymers other than polyvinyl alcohol, for example, proteins such as gelatin and casein; polysaccharides such as carboxymethyl cellulose, methyl cellulose, gum arabic, and alginic acid; synthetic polymers such as polyacrylic acid and ethylene-maleic anhydride copolymer, are used, the desired impermeable wall membrane cannot be obtained even when the aldehyde treatment according to the present invention is carried out.

Any core substance of a microcapsule which is substantially water-insoluble can be utilized as the core substance of the present invention. Examples of such substances are perfumes or flavors, animal and vegetable oils and fats, hydrocarbons, halogenated hydrocarbons, alcohols having 5 or more carbon atoms, aldehydes, esters, ethers, fatty acids, silicone oils, vitamins A, D, E, F, K, U and derivatives thereof, pharmacologically active substances such as galenicals and drugs, metal powders, metal oxides, adhesives, catalysts, coloring agents, and plastics.

Pectin is naturally widely present as a cell-constituting component of a plant body such as fruit and vegetables, and is particularly contained in a large amount in the rind of citrus fruit and apples. Thus, pectin is industrially advantageously obtained from the rind of such citrus fruit and apples. Furthermore, quince seed gum is a pectin contained in the flesh of plants represented by Cydonia oblonga belonging to a family of rose, can be used. Especially, the use of lemon pectin and apple pectin is preferable.

According to the present invention, an aqueous dispersion containing a microcapsule having a polyvinyl alcohol as a wall substance, and an aqueous solution containing 8% to 50% by weight of an electrolyte, is prepared.

In the present invention, any electrolyte capable of causing a phase separation of the polyvinyl alcohol in an acidic aqueous solution can be utilized. Any conventional inorganic and organic electrolytes may be used alone or in any mixture thereof.

The inorganic salts include acids, alkalis and the salts thereof, for example, water-soluble metal salts and ammonium salts of inorganic acids such as sulfuric acid, sulfurous acid, hydrochloric acid, phosphoric acid, metaphosphoric acid, boric acid, and nitric acid.

The organic salts include, for example, water-soluble metal salts and ammonium salts of organic acids.

Preferably, electrolytes having a high ionization degree and a high electric charge are used. More preferably, sulfates such as sodium sulfate, potassium sulfate, magnesium sulfate, aluminum sulfate, ammonium sulfate, and alum; chlorides such as sodium chloride, potassium chloride, calcium chloride, and ammonium chloride; and phosphates such as sodium hydrogen phosphate, potassium hydrogen phosphate, and ammonium phosphates, are used.

Preferably, the concentration of the electrolyte is 8% to 50% by weight, more preferably 10% to 40% by weight. When the amount is less than 8% by weight, the capsule membrane is dissolved or deformed in water and acceptable capsules cannot be obtained. Conversely, when the amount is more than 50% by weight, the electrolyte is precipitated and thus wastefully used. The term "concentration of the electrolyte" as used in the present invention denotes a concentration of the electrolyte in the aqueous dispersion. Namely, the concentration of the electrolyte = [electrolyte/(water + electrolyte)] x 100.

According to the present invention, the micro-capsule can be obtained by reacting the above-mentioned aqueous dispersion containing capsules with an aldehyde having a solubility in water at 20°C of not more than 10%.

Any aldehyde having a solubility in water at 20°C of 10% or less, preferably 0.001% to 7%, can be used. These aldehydes are specifically reacted with the polyvinyl alcohol, whereby the polyvinyl alcohol is hydrophobically treated without crosslinking.

Examples of such aldehydes are isobutylaldehyde, hexylaldehyde, heptylaldehyde, citral, benzaldehyde, salicylaldehyde, m- and p-hydroxybenzaldehyde, o-, m-, and p-methoxybenzaldehyde, o-, m-, and p-ethoxybenzaldehyde, veratraldehyde, vanillin, phenylpropyl aldehyde, phenylacetaldehyde, cinnamic aldehyde, and o-, m-, and p-methylbenzaldehyde.

The above-mentioned aldehydes have a low reactivity to hydrophilic polymers, other than the polyvinyl alcohol, for example, gelatin, carboxymethyl cellulose, gum arabic, alginic acid, and the polyacrylic acid content is low or is not substantially reacted therewith. Even if the reaction occurs, a capsule membrane exhibiting a desired impermeability cannot be obtained.

Aldehydes having a solubility in water at 20°C of more than 10%, for example, formaldehyde, glyoxal, and glutaraldehyde, have a high reactivity to polyvinyl alcohol, and although the polyvinyl alcohol is crosslinked to be insolubilized in water, the polyvinyl alcohol molecule itself is not insolubilized in water and a number of hydrophilic groups (OH groups) remains in the molecule. As a result, the resultant capsule is swollen in the aqueous solution and, in particular, a high impermeability against the core substance in the presence of a surfactant cannot be realized. Furthermore, aldehydes having a solubility in water at 20°C of more than 10% are extremely toxic to the human body.

Preferably, the aldehydes are added in an amount of 0.3 to 10 times, more preferably 0.3 to 5 times, of the weight of the polyvinyl alcohol.

The hydrophobic treatment with the aldehyde is carried out under an acidic condition, preferably at a pH of 0.5 to 4.5, more preferably a pH of 1.0 to 3.5. When the pH is too low, the desired hydrophobic wall membrane cannot be obtained, and conversely, when the acidity is too weak, the reaction does not proceed as required. Further, if the pH is on the alkaline side, an unwanted reaction occurs and the formation of the desired impermeable wall membrane becomes difficult.

Although there are no critical limitations to the reaction condition, the hydrophobic treatment is preferably carried out at a reaction temperature of 10°C to 80°C, more preferably 20°C to 60°C. When the reaction temperature is too low, the reaction does not substantially occur. Contrary to this, when the reaction temperature is too high, the capsule membrane is softened and deformed and the desired impermeable wall membrane is not obtained. Although the preferable reaction time depends upon the kind

4

of aldehyde, the suitable reaction time is 1 to 100 hours, more preferably 3 to 60 hours.

The aldehydes having a solubility in water at 20°C of 10% or less are specifically reacted with polyvinyl alcohol, and thus the polyvinyl alcohol is made hydrophobic without crosslinking. The resultant capsule membrane does not swell in water, and therefore, the desired high impermeability of the core substance through the membrane can be obtained. Furthermore, the degree of hydropholicity of the capsule membrane and the degree of hydrophilicity (or compatibility) of the membrane and the hydrophobic core substance can be adjusted by varying the kind of aldehyde and the reactivity of the polyvinyl alcohol. Accordingly, the core substance is not permeated, and further, a specific substance can be selectively allowed to permeate through the membrane.

According to the present invention, the micro-capsules with a wall membrane having an improved impermeability in an aqueous solution can be prepared by adding a specific aldehyde to an aqueous dispersion containing a microcapsule having a wall membrane of polyvinyl alcohol, followed by reacting the mixture. The resultant capsule completely blocks permeation by the core substance and has a high safety factor with respect to the human body.

The microcapsules obtained by the present invention can be suitably and widely utilized for aqueous products including a surfactant having a strong permeability, for example, a shampoo, rinse, liquid heavy detergent, kitchen detergent, toilet detergent, bathtub detergent, toothpaste, mouth wash agent, and soap, and further, cosmetics such as a cream, lipstick, and lotion, pharmaceutical preparations such as a blennororrhea agent and blemish agent, adhesives, catalysts; and the like.

## EXAMPLES

The present invention will now be further illus trated by, but is by no means limited to, the following Examples, wherein "%" represents "% by weight" unless otherwise specified.

## Example 1

As a core material, 7 g of ℓ-menthol was dissolved in 20 g of 2-octyl dodecanol. Then 4.0 g of polyvinyl alcohol (average degree of polymerization 550, degree of saponification 88 mole%) and 0.3 g of pectin were dissolved in 95.7 g water, and 27 g of the core material was dispersed to adjust an average particle size thereof to about 500 μm.

To the dispersion obtained above, 100 g of 25% aqueous sodium chloride solution was dropwise added while stirring, at a temperature of 30°C, and microcapsules comprising the core material coated with a concentrated aqueous polyvinyl alcohol solution were obtained.

Thereafter, a mixture of 10 g of a 5% aqueous pectin solution and 60 g of a 25% aqueous sodium sulfate solution was added, followed by adding 30 g of sodium sulfate. As a result, 327 g of the following aqueous dispersion containing capsules having a wall membrane of polyvinyl alcohol dispersed therein was obtained.

| Pectin | 0.27% |
|---|---|
| Sodium chloride | 8.40% |
| Sodium sulfate | 15.20% |
| Water | Balance |

To the aqueous dispersion of the capsules obtained above, 10% acetic acid was added to adjust the pH to 2.5, and then 10 g of vanillin was added and the mixture was stirred at 40°C for 20 hours. Thereafter, the pH was changed to 1.5 and the reaction was carried out for 15 hours.

After the reaction, the capsules were separated by a 42 mesh sieve, followed by washing with water, and about 30 g of the capsules was obtained.

## Comparative Example 1

To the aqueous capsule dispersion, 10% acetic acid and an aqueous sodium hydroxide solution were added to adjust the pH to 3.5, and thereafter, 10 g of a 50% aqueous glutaraldehyde was added and a curing reaction was carried out at 10°C for 20 hours. After the reaction, the capsules were separated by a 42 mesh sieve, followed by washing with a 20% aqueous sodium sulfate solution, and about 30 g of the capsules was obtained.

## Comparative Example 2

As a core material, 7 g of l-menthol was dissolved in 20 g of 2-octyldodecanol. Then 27 g of the core material was dissolved in 56 g of a 10% aqueous solution of gelatin (isoelectric point 8.8, jelly strength 260 bloom), followed by adding 28 g of a 20% aqueous gum arabic solution to adjust the average particle size thereof to about 500 μm.

Thereafter, 5% acetic acid was dropwise added at 40°C to adjust the pH to 4.1, the dispersion was allowed to cool to 15°C, and capsules comprising the core material coated with the concentrated aqueous solution of gelatin-gum arabic were obtained.

A 4 g amount of 50% glutaraldehyde was then added, followed by effecting a curing reaction at 10°C for 20 hours, and after the curing reaction, the resultant capsules were separated by a 42 mesh sieve followed by washing with water, and about 30 g of the capsules was obtained.

## Evaluation Example 1

A 10 g amount each of the capsules obtained in Example 1 and Comparative Examples 1 and 2 was independently dispersed in 1000 g of a 5% aqueous sodium lauryl sulfate solution and stored at 50°C in a sealed glass bottle.

The aqueous dispersion was sampled at predetermined intervals, and after filtering through a filter paper, the concentration of l-menthol in the aqueous solution was determined by gas chromatography, and the rate of elution of the l-menthol from the capsule was calculated.

The results are shown in Table 1.

Table 1

| No. | Rate of eluation (%) of l-menthol | |
|---|---|---|
| | 14 days | 30 days |
| Example 1 | 1% | 2% |
| Comparative Example 1 | 55% | 85% |
| Comparative Example 2 | 60% | 90% |

As clear from the results shown in Table 1, the rate of eluation of l-menthol from the capsule of Example 1 was remarkably low and very little permeation of the substances through the wall membrane of the capsule occurred.

## Comparative Example 3

In Comparative Example 2, 5 g of vanillin and 20 g of sodium chloride were added, instead of 50% glutaraldehyde, and after adjusting the pH to 4.0 with 30% sulfuric acid, the mixture was warmed to 40°C followed by stirring for 30 hours.

When the resultant capsule was added to water at 80°C, the wall membrane of the capsule was dissolved.

## Comparative Example 4

The procedures of Comparative Example 3 were repeated, except that the pH was adjusted to 2.5.
When the resultant capsule was added to water at 80°C, the wall membrane of the capsule was dissolved.

## Comparative Example 5

In Comparative Example 4, o-methoxybenzaldehyde was used instead of vanillin, and when the resultant capsule was added to water at 80 °C, the wall membrane of the capsule was dissolved.

As clearly shown in Comparative Examples 3 to 5, the desired water-resistant wall membrane cannot be obtained in capsules having a wall membrane composed mainly of gelatin, even when the capsule is treated with aldehydes having a solubility in water at 20 °C of 10% or less.

## Example 2

To the aqueous dispersion of Example 1, 30% sulfuric acid was added to adjust the pH to 1.5, the 5 g of o-methoxybenzaldehyde was added and a curing reaction was carried out at 40 °C for 15 hours.

After the reaction, the capsules were separated by a 42 mesh sieve followed by washing with a 20% aqueous sodium sulfate solution, and about 30 g of the capsules was obtained.

## Evaluation Example 2

The capsules of Example 2 and Comparative Examples 1 and 2 were independently formulated at a concentration of 1% in the following toothpaste compositions. The resultant toothpaste compositions were filled in laminate tubes and stored at 40 °C.

| Composition of Toothpaste | |
|---|---|
| Ingredient | % |
| Silica anhydride | 15.0 |
| Sodium carboxymethyl cellulose | 1.0 |
| Propylene glycol | 2.0 |
| Sorbitol | 45.0 |
| Sodium lauryl sulfate | 1.0 |
| Saccharin sodium | 0.1 |
| Flavor | 0.7 |
| Water | Balance |

A 2 g amount of the toothpaste was sampled at predetermined intervals and the capsules separated by a 42 mesh sieve after diluting five-fold with water. A 5 g amount of the dispersion permeated through the sieve was taken, and after adding 2 g of n-amyl alcohol thereto, the mixture was shaken and $l$-menthol eluated into the toothpaste was extracted. The concentration of the $l$-menthol was determined by gas chromatography and the ratio of $l$-menthol eluated into the toothpaste from the capsule was calculated.

The results are shown in Table 2.

Table 2

| No. | Rate of eluation (%) of $l$-menthol | |
|---|---|---|
| | 14 days | 30 days |
| Example 2 | 1% | 2% |
| Comparative Example 1 | 60% | 90% |
| Comparative Example 2 | 70% | 95% |

As clear from the results shown in Table 2, the permeability of the wall membrane of the capsules of Example 2 is remarkably lower than those of the Comparative Examples 2 and 3.

Example 3

As a core material, 2 g of Vitamin C stearate was dispersed in 100 g of linolenic acid. Then 17.5 g of polyvinyl alcohol (average degree of polymerization 1000, degree of saponification 88 mole%) and 1.3 g of pectin were dissolved in 400 g of water, and 100 g of the core material was dispersed to adjust the average particle size thereof to about 100 μm.

To the dispersion obtained above, 400 g of a 25% aqueous sodium sulfate solution was dropwise added, followed by adding 40 g of a 5% aqueous pectin solution, 250 g of a 25% aqueous sodium sulfate solution, and 60 g of a 5% aqueous pectin solution.

Accordingly, an aqueous dispersion containing capsules having a strong wall membrane of polyvinyl alcohol was obtained without agglomeration of the capsules.

To the above-prepared aqueous dispersion containing capsules was added 10% hydrochloric acid, to adjust the pH to 2.0, and thereafter, 30 g of heptylaldehyde was added followed by stirring at 50°C for 20 hours. The capsules were then separated and washed with water, and then stored in a 15% aqueous sodium sulfate solution. The wall membrane of the capsules thus obtained possessed a high water resistance such that the wall membrane was not dissolved in water at 100°C.

The capsules obtained above were suitable for use in the formulation of a skin cream as a vitamin C providing agent and emollient agent.

Example 4

As a core material, 4 g of benzyl alcohol, 4 g of linalool, 5 g of eugenol, and 15 g of isostearyl alcohol were mixed. Then 4 g of polyvinyl alcohol (average degree of polymerization 500, degree of saponification 87 - 89 mole%) and 0.3 g of pectin were dissolved in water to a total amount of 100 g, and then 28 g of the above-prepared core material was dispersed therein to adjust an average particle size thereof to about 500 μm.

To the dispersion obtained above, a mixture of 51 g of a 25% aqueous sodium chloride solution and 6.5 g of a 5% aqueous pectin solution was dropwise added while stirring, at a temperature at 30°C. Then a mixture of 46 g of a 25% aqueous sodium chloride solution and 12.5 g of a 5% aqueous pectin solution was dropwise added, followed by adding 60 g of a 25% aqueous sodium sulfate solution.

Accordingly, acceptable capsules were obtained without agglomeration of the capsules.

The pH of the aqueous dispersion of the capsules was adjusted to 3.5 with 10% sodium hydroxide, and then 7 g of phenylpropyl aldehyde was added thereto, followed by stirring at 40°C for 15 hours. Thereafter, the pH of the aqueous dispersion was adjusted to 1.5 with 10% sulfuric acid, followed by stirring at 40°C for 20 hours.

The capsules obtained above were centrifugally separated and washed with water, and it was found that the wall membrane of the resultant capsule was stable and was neither swollen nor dissolved in water at 100°C. The resultant capsules were suitable for use in the formulation of toothpaste.

Example 5

In Example 4, p-methylbenzaldehyde was used instead of the phenylpropyl aldehyde. The wall membrane of the resultant capsules was stable and was neither swollen nor dissolved in water at 100°C.

Example 6

In Example 4, cinnamic aldehyde was used instead of the phenylpropyl aldehyde. The wall membrane of the resultant capsules was stable and was neither swollen nor dissolved in water at 100°C.

Example 7

8

As a core material, 100 g of methylphenyl silicone, 100 g of liquid paraffin, and 50 g of squalane were mixed.

Then a 5% aqueous solution of polyvinyl alcohol (degree of polymerization 1500, degree of saponification 98.5%) was charged to an outer cylinder of a concentric double orifice (diameter of inner cylinder 0.5 mm, diameter of outer cylinder 2 mm) and was dropwise added to a 1% aqueous borax solution, and a capsule having a wall membrane of polyvinyl alcohol was prepared. After drying, capsules having a particle size of 3 to 5 mm was obtained.

A 30 g amount of the capsules obtained above was dispersed in 200 g of a 25% aqueous sodium sulfate solution, and then 10 g of 5% pectin was added thereto followed by adjusting the pH with 20% sulfuric acid to 1.5. Thereafter, 5 g of o-methoxybenzayldehyde was added, and after stirring at 40°C for 20 hours, the resultant capsules were separated and washed with water. The resultant capsules were stable and were neither swollen nor dissolved at 100°C in water. The capsules were suitable for use in the formulation of a hair rinse.

Evaluation Example 3

The capsules obtained in Examples 3 to 7 were evaluated in the same manner as in Evaluation Example 1.

The results are shown in Table 3.

Table 3

| Example No. | Analyzed core component | Rate of eluation (%) | |
|---|---|---|---|
| | | 14 days | 30 days |
| 3 | linolenic acid | 0.5 | 1 |
| 4 | benzyl alcohol | 1.5 | 3 |
| 5 | " | 1 | 2.5 |
| 6 | " | 2 | 3.5 |
| 7 | squalane | 0 | 0.5 |

## Claims

1. A method of producing microcapsules comprising the steps of:
mixing (i) an aqueous dispersion containing capsules having a wall membrane containing polyvinyl alcohol and an aqueous solution containing 8% to 50% by weight of an electrolyte or electrolytes and (ii) an aldehyde or aldehydes having a solubility in water at 20°C of not more than 10%; and allowing the mixture to react under an acidic condition.

2. A method as claimed in claim 1, wherein the polyvinyl alcohol contains at least 50% by weight of a vinyl alcohol component in the polymer.

3. A method as claimed in claim 1, wherein said electrolyte is at least one member selected from the group consisting of inorganic salts and organic salts.

4. A method as claimed in claim 1, wherein a concentration of the electrolyte in the aqueous solution thereof is 10% to 40% by weight.

5. A method as claimed in claim 1, wherein said aldehyde is at least one member selected from the group consisting of isobutylaldehyde, hexylaldehyde, heptylaldehyde, citral, benzaldehyde, salicylaldehyde, m- and p-hydroxybenzaldehyde, o-, m-, and p-methoxybenzaldehyde, o-, m-, and p-ethoxybenzaldehyde, veratraldehyde, vanillin, phenylpropyl aldehyde, phenylacetaldehyde, cinnamic aldehyde, and o-, m-, and p-methylbenzaldehyde.

6. A method as claimed in claim 1, wherein the amount of the aldehyde added is 0.3 to 10 times of the weight of the polyvinyl alcohol.

7. A method as claimed in claim 1, wherein the acidic condition is a pH of 0.5 to 4.5.

8. A method as claimed in claim 1, wherein pectin is added simultaneously with or after mixing of the electrolyte.

9. A method as claimed in claim 8, wherein the amount of the pectin is 0.1% to 1.0% by weight based upon the amount of the water.